# EUROPEAN PATENT SPECIFICATION

(11) **EP 1 543 139 B1**
(45) Date of publication and mention of the grant of the patent: **13.06.2007**
(21) Application number: 03748056.3
(22) Date of filing: 18.09.2003
(51) Int. Cl.: C12P 17/12, C12N 1/21, C12N 9/02

(54) **RECOMBINANT MICROORGANISM FOR THE PRODUCTION OF VITAMIN B6**
REKOMBINANTE MIKROORGANISMEN FÜR DIE PRODUKTION VON VITAMIN B6
MICRO-ORGANISME RECOMBINANT POUR LA PRODUCTION DE VITAMINE B6

(30) Priority: 27.09.2002 EP 02021623
(43) Date of publication of application: 22.06.2005
(73) Proprietor: DSM IP Assets B.V., 2611 TE Heerlen (NL)
(72) Inventor: HOSHINO, Tatsuo, Kanagawa-ken, Kanagawa 248-0027 (JP); ICHIKAWA, Keiko, Marin-terasu-nibankan 201, Kanagawa-ken, Kanagawa 251-0047 (JP); TAZOE, Masaaki, 5-14-22 Yokodai, Kanagawa-ken, Kanagawa 235-0045 (JP)
(74) Representative: Keller, Günter
(86) International application number: PCT/EP2003/010403
(87) International publication number: WO 2004/029271

(56) References cited:
- EP-A- 0 950 715
- YANG YONG ET AL: "Involvement of the gapA- and epd (gapB)-encoded dehydrogenases in pyridoxal 5'-phosphate coenzyme biosynthesis in Escherichia coli K-12" JOURNAL OF BACTERIOLOGY, vol. 180, no. 16, August 1998 (1998-08), pages 4294-4299, XP002266176 ISSN: 0021-9193
- MITTENHUBER GERHARD: "Phylogenetic analyses and comparative genomics of vitamin B6 (pyridoxine) and pyridoxal phosphate biosynthesis pathways" JOURNAL OF MOLECULAR MICROBIOLOGY AND BIOTECHNOLOGY, vol. 3, no. 1, January 2001 (2001-01), pages 1-20, XP008026190 ISSN: 1464-1801
- ZHAO GENSHI ET AL: "An Escherichia coli K-12 tktA tktB mutant deficient in transketolase activity requires pyridoxine (vitamin B-6) as well as the aromatic amino acids and vitamins for growth" JOURNAL OF BACTERIOLOGY, vol. 176, no. 19, 1994, pages 6134-6138, XP002266177 ISSN: 0021-9193
- FRANCO M G ET AL: "STRUCTURAL BASIS FOR THE FUNCTION OF PYRIDOXINE 5'-PHOSPHATE SYNTHASE" STRUCTURE, CURRENT BIOLOGY LTD., PHILADELPHIA, PA, US, vol. 9, no. 3, March 2001 (2001-03), pages 245-253, XP001005211 ISSN: 0969-2126
- MARTENS J-H ET AL: "MICROBIAL PRODUCTION OF VITAMIN B12" APPLIED MICROBIOLOGY AND BIOTECHNOLOGY, SPRINGER VERLAG, BERLIN, DE, vol. 58, 2002, pages 275-285, XP001164113 ISSN: 0175-7598

### SEQUENCE LISTING

<110> Roche Vitamins AG
<120> Recombinant microorganism for the production of vitamin B6
<130> NDR5214
<160> 12
<170> PatentIn version 3.1
<210> 1
   <211> 20
   <212> DNA
   <213> Artificial
<400> 1
   cctgcaggca ggagatctat 20
<210> 2
   <211> 20
   <212> DNA
   <213> Artificial
<400> 2
   cctgcagacg ctgcttgcgt 20
<210> 3
   <211> 20
   <212> DNA
   <213> Artificial
<400> 3
   tcccgggagg ggaaatggct 20
<210> 4
   <211> 20
   <212> DNA
   <213> Artificial
<400> 4
   acccgggcaa aatttcggca 20
<210> 5
   <211> 29
   <212> DNA
   <213> Artificial
<400> 5
   ccgaattcag gcccctgatg agttttgat 29
<210> 6
   <211> 29
   <212> DNA
   <213> Artificial
<400> 6
   ccgaattcag gagtggagta gggattatg 29
<210> 7
   <211> 20
   <212> DNA
   <213> Artificial
<400> 7
   ggaattcagg taacacaaac 20
<210> 8
   <211> 20
   <212> DNA
   <213> Artificial
<400> 8
   ggaattcatg aagaagagat 20
<210> 9
   <211> 20
   <212> DNA
   <213> Artificial
<400> 9
   gaagcttgat gaggattgtc 20
<210> 10
   <211> 20
   <212> DNA
   <213> Artificial
<400> 10
   taagcttgcc attagccacg 20
<210> 11
   <211> 24
   <212> DNA
   <213> Artificial
<400> 11
   caattgatgg ttaaaaccca acgt 24
<210> 12
   <211> 24
   <212> DNA
   <213> Artificial
<400> 12
   gaattctcat tgggtgttaa caat 24

### SEQUENCE LISTING

<110> Roche Vitamins AG
<120> Recombinant microorganism for the production of vitamin B6
<130> NDR5214
<140> PCT/EP03/10403
   <141> 2003-09-18
<150> EP 02021623.0
   <151> 2002-09-27
<160> 12
<170> PatentIn version 3.1
<210> 1
   <211> 20
   <212> DNA
   <213> Artificial sequence
<220>
   <223> Primer 1 for amplifying the epd gene
<400> 1
   cctgcaggca ggagatctat 20
<210> 2
   <211> 20
   <212> DNA
   <213> Artificial sequence
<220>
   <223> Primer 2 for amplifying the epd gene
<400> 2
   cctgcagacg ctgcttgcgt 20
<210> 3
   <211> 20
   <212> DNA
   <213> Artificial Sequence
<220>
   <223> Primer 1 for amplifying the serC gene
<400> 3
   tcccgggagg ggaaatggct 20
<210> 4
   <211> 20
   <212> DNA
   <213> Artificial sequence
<220>
   <223> Primer 2 for amplifying the serC gene
<400> 4
   acccgggcaa aatttcggca 20
<210> 5
   <211> 29
   <212> DNA
   <213> Artificial sequence
<220>
   <223> Primer 1 for amplifying the dxs gene
<400> 5
   ccgaattcag gcccctgatg agttttgat 29
<210> 6
   <211> 29
   <212> DNA
   <213> Artificial Sequence
<220>
   <223> Primer 2 for amplifying the dxs gene
<400> 6
   ccgaattcag gagtggagta gggattatg 29
<210> 7
   <211> 20
   <212> DNA
   <213> Artificial Sequence
<220>
   <223> Primer 1 for amplifying the pdxB gene
<400> 7
   ggaattcagg taacacaaac 20
<210> 8
   <211> 20
   <212> DNA
   <213> Artificial Sequence
<220>
   <223> Primer 2 for amplifying the pdxB gene
<400> 8
   ggaattcatg aagaagagat 20
<210> 9
   <211> 20
   <212> DNA
   <213> Artificial sequence
<220>
   <223> Primer 1 for amplifying the pdxJ gene
<400> 9
   gaagcttgat gaggattgtc 20
<210> 10
   <211> 20
   <212> DNA
   <213> Artificial sequence
<220>
   <223> Primer 2 for amplifying the pdxJ gene
<400> 10
   taagcttgcc attagccacg 20
<210> 11
   <211> 24
   <212> DNA
   <213> Artificial Sequence
<220>
   <223> Primer 1 for amplifying the pdxA gene
<400> 11
   caattgatgg ttaaaaccca acgt 24
<210> 12
   <211> 24
   <212> DNA
   <213> Artificial Sequence
<220>
   <223> Primer 2 for amplifying the pdxA gene
<400> 12
   gaattctcat tgggtgttaa caat 24

## Description

The present invention relates to a recombinant microorganism and a process for preparing vitamin B₆ by using the same.

"Vitamin B₆" as used in the present invention includes pyridoxol, pyridoxal, and pyridoxamine. Vitamin B₆ is a vitamin indispensable to human beings or other animals and used as a raw material of medicines or as feed additives.

The present invention provides recombinant microorganisms carrying the cloned genes for over-expression of the enzymes involved in the vitamin B₆ biosynthetic pathway to produce vitamin B₆.

Examples for suitable microorganisms include members of the genus *Escherichia,* capable of over-producing vitamin B₆, e.g. *E. coli* AT1024 or WG497.

The present invention also provides a method to prepare vitamin B₆ effectively by cultivating said recombinant microorganism resulting in accumulation of multipliable amounts of vitamin B₆ in culture broth and to separate vitamin B₆ from the culture broth.

The present invention also provides a method for preparing vitamin B₆ by cultivating a recombinant microorganism, which is expressing the cloned genes encoding enzymes involved in the vitamin B₆ biosynthetic pathway, in appropriate medium, and by separating vitamin B₆ from the culture broth.

Preferably the recombinant microorganism carries an extra nucleic acid encoding an enzyme or an enzyme combination selected from
i) erythrose 4-phosphate dehydrogenase (E4P dehydrogenase),
ii) E4P dehydrogenase and 1-deoxy-D-xylulose 5-phosphate synthase (DXP synthase),
iii) E4P dehydrogenase and pyridoxol 5'-phosphate synthase (PNP synthase), and
iv) E4P dehydrogenase, DXP synthase and PNP synthase.

In the vitamin B₆ biosynthesis in *E. coli* PNP (pyridoxol 5'-phophate) is synthesized de novo by the condensation of two intermediates, 4PHT (4-phosphohydroxy-L-threonine) and DXP (1-deoxy-D-xylulose-5-phophate), catalyzed by 4PHT dehydrogenase and PNP synthase. 4PHT is synthesized via a pathway leading from E4P (erythrose 4-phosphate) by a three-step reaction: dehydrogenation of E4P by E4P dehydrogenase; dehydrogenation of 4-phospho-erythronate catalyzed by 4-phospho-erythronate dehydrogenase; and amination of 3-hydroxy-4-phosphohydroxy-α-ketobutyrate catalyzed by phosphoserine aminotransferase. DXP is synthesized by a condensation of glyceraldehyde 3-phosphate and pyruvate with DXP synthase.

In these pathways, any step can be a bottleneck. In developing an industrial process for producing vitamin B₆, the amount of the vitamin produced can be increased, e.g., by overcoming the bottlenecks. This can be achieved by, e.g., increasing the amount of enzyme on the reaction. The present invention also relates to the cloning and over-expressing of genes coding for enzymes involved in the vitamin B₆ pathway in a host cell. Increasing the amount of an enzyme can be achieved, e.g., by introduction of desired genes in expressible form, e.g., using vectors replicable in a host cell, and additionally (i) by increasing the intended gene copy number, e.g., using a plasmid with an increased copy number, and/or (ii) by increasing transcription, e.g., by using promoter elements, and/or (iii) by increasing the rate of translation, e.g., by using a consensus ribosome binding site. Combinations of additional (i), (ii), and/or (iii) can also be employed for increasing the amount of an enzyme involved in the vitamin B₆ biosynthetic pathway.

In a preferred embodiment of the invention, more than one gene of the vitamin B₆ biosynthetic pathway was introduced into the microorganisms.

The appropriate genes may be isolated in accordance with known methods. The complete nucleotide sequences of *E. coli* K-12 (ATCC 25404) are known [Blattner et al., Science 277:1453-1474 (1997)] and deposited in databases. When the *E. coli* genes relevant to vitamin B₆ biosynthesis are being cloned, the polymerase chain-reaction (PCR) method is suitable.

For PCR, at the 5' end of the gene, primers are designed to amplify the entire protein-coding-region including the initiation codon and the ribosome binding site preceding it. At the 3' ends of the genes, the codons responsible for termination of protein synthesis are always included in the amplified segment, but any transcription termination signals are excluded. All primers are modified by addition of the recognition sequences for specific restriction endonucleases at the 5' end of each primer.

According to the present invention, enhanced expression of genes coding for enzymes responsible for biosynthesis of vitamin B₆ is mediated by transferring the corresponding genes into a host cell in expressible form. The genes may be chromosomal (e.g. integrated into a host cell chromosome by homologous recombination or other mechanism) or extrachromosomal (e.g. carried by plasmids, cosmids, phages and the like).

The genes can be introduced into a host cell by plasmids, cosmids, phages, or other vectors that mediate transfer of genes into a host cell. Selectable markers can be present on the vector to aid in identification of host cells into which the genes have been introduced. Examples for selectable markers are genes that confer resistance to particular antibiotics, such as tetracycline, ampicillin, chloramphenicol, kanamycin, or neomycin (referred to as Tc, Ap, Cm, Km, and Nm, respectively, hereinafter).

A means for introducing genes into a host cell may use an extrachromosomal multi-copy plasmid vector into which genes have been inserted. Plasmid-borne introduction of the genes into host cells involves an initial cleaving of a plasmid with a restriction enzyme, followed by ligation of the plasmid and genes. Upon recircularization of the ligated recombinant plasmid, transfer into the host cell may be carried out by methods well known in the art such as electroporation, calcium-dependent transformation, and conjugation. Plasmids suitable for insertion of genes into the host cell include, but are not limited to, pBR322 and its derivatives such as pKK223-3, pUC vectors, and pACYC and its derivatives such as pSTV29. In addition, cosmid vectors such as pVK100 are also suitable for the insertion of the genes into host cells.

The amplified gene can be placed on a vector capable of being expressed in a host cell, and can be transformed into a microorganism producing vitamin B₆ in accordance with current methods. In the case of introducing the other gene into a microorganism as the second gene in this invention, the gene can be placed on the other vector, which is compatible to the present plasmid in the host cell, and can be transformed into a microorganism which already carries the first plasmid.

Thus-obtained microorganisms may be cultivated in a medium containing assimilable carbon sources, digestible nitrogen sources, inorganic salts and other nutrients necessary for growth of the microorganism. As the carbon source, e.g., glucose, fructose, lactose, galactose, sucrose, maltose, starch, dextrin or glycerol may be employed. As the nitrogen source, e.g., peptone, soybean powder, corn steep liquor, yeast extract, meat extract, ammonium sulfate, ammonium nitrate or mixtures thereof may be employed. Inorganic salts, sulfates, hydrochlorides or phosphates of calcium, magnesium, zinc, manganese, cobalt and iron may be employed. Conventional nutrient factors or an antifoaming agent such as animal oil, vegetable oil or mineral oil can also be present. The pH of the culture medium is suitably in a range of from about 5.0 to about 9.0, preferably 6.5 to 7.5. The cultivation temperature is suitably in a range of from about 10°C to 40°C, preferably 34°C to 37°C. The cultivation time is suitably about 1 day to 7 days, preferably 2 days to 3 days. In the cultivation, aeration and agitation usually give favorable results.

The amount of vitamin B₆ produced in culture broth can be assayed by the turbidity method with *Saccharomyces carlsbergensis* ATCC 9080. Vitamin B₆ derivatives such as pyridoxol, pyridoxal, and pyridoxamine can be separately quantified by high pressure liquid chromatography (referred to as HPLC hereinafter).

Vitamin B₆ can be collected from the culture broth, e.g., by separating and removing the cells, subjecting to ion exchange resin treatment, concentration cooling crystallization, membrane separation, and other known methods in any suitable combination. In order to remove impurities, activated carbon adsorption and re-crystallization may be used for purification.

The invention is explained in more detail below with the aid of a few implementation examples.

### General methods

*E. coli* AT1024 is freely available from CGSC under number 4559.
In the genetic studies, strains of *E. coli* were, unless otherwise indicated, cultured on LB medium consisting of 1 % Bacto Tryptone (Becton Dickinson Microbiology systems, MD, USA), 0.5 % Bacto Yeast extract (Becton Dickinson Microbiology systems, MD, USA) and 0.5 % NaCl. Depending on the resistance properties of the strains employed, Ap (100 µg/ml), Cm (100 µg/ml), Tc (10 µg/ml) or a mixture thereof was added to the medium if necessary. For this, Ap was dissolved in water, Tc in 50% ethanol, Cm in ethanol, and the solutions were added, after having been sterilized by filtration, to the previously autoclaved medium. Bacto-agar (1.5%) was added to the LB medium for preparing agar plates. Plasmid DNA was isolated from *E. coli* with QIAGEN Midi kit (QIAGEN GmbH, Germany) or with Automatic DNA Isolation System PI-50 (Kurabo Industry Ltd., Japan). Chromosomal DNA was isolated from *E*. *coli* K-12 using QIAGEN genomic-tips (QIAGEN GmbH, Germany).

Restriction enzymes, alkaline phosphatase, ligation kit (Takara Bio. Inc, Shiga, Japan), TOPO TA cloning kit (Invitrogen Japan K.K., Japan); and KOD Dash (Toyobo Co., Ltd., Japan) were used in accordance with the producers' instructions. For restriction enzyme analysis, the DNA fragments were fractionated in agarose gels (1.0%) and isolated from the gels by means of extraction using a commercially available system with QIAEXII (QIAGEN GmbH, Germany). DNA sequence was determined with an ALF DNA sequencer (Amersham Biosciences Corp., NJ, USA).

For transformation, the cells were incubated with a shaking of 160 rpm at 37°C for 2.5-3 h in LB medium (50 ml in 200-ml flasks). At an optical density (600 nm) of approx. 0.4, the cells were spun down and taken up in one tenth the volume of 0.1M MgCl₂. After an incubation of 30 min at 4°C with from 0.1 to 100 ng of DNA, and subsequent incubation at 37°C for 1 hour, the cells were plated out on LB medium containing appropriate antibiotics.

### Example 1: Construction of recombinant plasmid pKK-epd

The epd gene was amplified from 100 ng of chromosomal DNA of *E. coli* K-12 with advantage-HF PCR kit using 10-pmol of two primers, SEQ ID NO:1 and 2. Reaction conditions were as follows; after holding 15 sec at 94°C, 25 cycles of 15 sec at 94°C, 3 min at 68°C. The amplified 1.0-kb fragment was directly cloned in pCRII-TOPO vector with TOPO TA cloning kit. Sequence of amplified region was ascertained to be identical with the CDS region of epd (3070692-3071711, complement) in accession number NC_000913. The DNA corresponding to amplified region was cut out by digestion with PstI and recovered from agarose gel. This DNA was ligated to pKK223-3 expression vector (Amersham Biosciences Corp., NJ, USA) that has been opened with PstI and dephospharylated. After transformation of E. coli JM109 competent cells (Takara Bio. Inc, Shiga, Japan), plasmids of transformants were prepared and analyzed with restriction enzyme. A recombinant plasmid pKK-epd, wherein epd gene was inserted into the PstI site of pKK223-3 as the same direction of tac promoter on the vector, was obtained. Plasmid pKK-epd was prepared with E. coli JM109/pKK-epd with QIAGEN Midi kit.

### Example 2: Construction of recombinant plasmid pKK-serC

The serC gene of *E*. *coli* K-12 was amplified as described in Example 1, except using two primers, SEQ ID NO:3 and 4. The resulting 1.1-kb PCR product was inserted into pCRII-TOPO. It was ascertained that the sequence of the amplified region was identical with the CDS region of serC (956876-957964) in accession number NC_000913. The DNA corresponding to the amplified region was inserted into pKK223-3 yielding pKK-serC as in Example 1 except for digestion with SmaI. Plasmid pKK-serC was prepared with E. coli JM109/pKK-serC with QIAGEN Midi kit.

### Example 3: Construction of recombinant plasmid pKK-dxs

The dxs gene of *E. coli* K-12 was amplified as in Example 1, except using two primers, SEQ ID NO:5 and 6. The resulting 1.9-kb PCR product was inserted into pCRII-TOPO, and the sequence of the amplified region was ascertained to be identical with the CDS region of dxs (437539-439401, complement) in accession number NC_000913. The DNA corresponding to amplified region was inserted into pKK223-3 yielding pKK-dxs as in Example 1 except for digestion with EcoRI. Plasmid pKK-dxs was prepared with E. coli JM109/pKK-dxs with QIAGEN Midi kit.

### Example 4: Construction of recombinant plasmid pKK-pdxB

The pdxB gene of *E. coli* K-12 was amplified as in Example 1 except of using two primers, SEQ ID NO:7 and 8. Reaction conditions were as follows; after holding 15 sec at 94°C, 25 cycles of 15 sec at 94°C, 1 min at 58°C, 1 min at 72°C. The resulting 1.15-kb PCR product was inserted into pCRII-TOPO, and sequence of amplified region was ascertained to be identical with the CDS region of pdxB (2434735-2435871, complement) in accession number NC_000913. The DNA corresponding to amplified region was inserted into pKK223-3 yielding pKK-pdxB as in Example 1 except for digestion with EcoRI. Plasmid pKK-pdxB was prepared from *E. coli* JM109/pKK-pdxB with QIAGEN Midi kit.

### Example 5: Construction of recombinant plasmid pKK-pdxJ

The pdxJ gene of *E*. *coli* K-12 was amplified as the same procedure as described in Example 4, except using two primers, SEQ ID NO: 9 and 10. The resulting 0.75-kb PCR product was inserted into pCRII-TOPO, and that sequence of amplified region was ascertained to be identical with the CDS region of pdxJ (2699018-2699749, complement) in accession number NC_000913. The DNA corresponding to amplified region was inserted into pKK223-3 yielding pKK-pdxJ as in Example 1 except for digestion with HindIII. Plasmid pKK-pdxJ was prepared with E. coli JM109/pKK-pdxJ with QIAGEN Midi kit.

### Example 6: Construction of recombinant plasmid pKK-pdxA

The pdxA gene of *E. coli* K-12 was amplified by PCR from 100 ng of chromosomal DNA of *E. coli* K-12 with KOD Dash using 10-pmol of two primers, SEQ ID NO: 11 and 12. Reaction conditions were as follows; after holding 1 min at 94°C, 30 cycles of 30 sec at 94°C, 2 sec at 48°C, 30 sec at 74°C. The resulting 1.0-kb PCR product was inserted into pCRII-TOPO, and that sequence of amplified region was ascertained to be identical with the CDS region of pdxA (52427-53416, complement) in accession number NC_000913. The DNA corresponding to amplified region was cut out by double digestion with MunI and EcoRI and recovered from agarose gel. The DNA fragment was inserted into pKK223-3 yielding pKK-pdxA as in Example 1 except for digestion with EcoRI. Plasmid pKK-pdxA was prepared with E. coli JM109/pKK-pdxA with QIAGEN Midi kit.

### Example 7: Construction of recombinant plasmid pVK-pdxJ

Plasmid pKK-pdxJ obtained in Example 5 was digested with ScaI and SphI. Resulting 2-kb fragment containing tac promoter and pdxJ was purified from agarose gel. Plasmid pVK100 was prepared from *E. coli* HB101/pVK100, digested with BglII, blunt-ended with blunting kit and dephosphorylated. The 2-kb fragment was ligated to thus-obtained pVK100. *E. coli* HB101 competent cells (Takara Bio Inc., Shiga, Japan) were transformed with this ligation mixture and plasmids of transformants were analyzed with restriction enzyme. A recombinant plasmid pVK-pdxJ, wherein tac promoter and pdxJ gene were inserted into the BglII site of pVK100 as the same direction of Km resistant gene, was obtained. Plasmid pVK-pdxJ was prepared with *E. coli* HB101/pVK-pdxJ with QIAGEN Midi kit.

### Example 8: Construction of recombinant plasmid pVK-dxs

Plasmid pKK-dxs obtained in Example 3 was digested with BamHI and resulting 2.2-kb fragment containing tac promoter and dxs was purified from agarose gel. Plasmid pVK100 was cleaved with BglII and dephosphorylated. The 2.2-kb fragment was ligated to thus-obtained pVK100 yielding pVK-dxs, wherein tac promoter and dxs gene were inserted into the BglII site of pVK 100 as the same direction of Km resistant gene, as in Example 7. Plasmid pVK-dxs was prepared with *E*. *coli* HB101/pVK-dxs with QIAGEN Midi kit.

### Example 9: Construction of recombinant plasmid pSTV-dxs

The 2.2-kb fragment obtained in Example 8 was ligated to pSTV29 (TaKaRa Bio Inc., Japan) that had been cleaved with BamHI and dephosphorylated. A recombinant plasmid pSTV-dxs, wherein tac promoter and dxs gene were inserted into the BamHI site of pSTV29 as the same direction of lacZ gene, was obtained as in Example 7. Plasmid pSTV-dxs was prepared with *E. coli* HB101/pSTV-dxs with QIAGEN Midi kit.

### Example 10: Preparation of microorganisms harboring recombinant plasmids

*E. coli* AT1024 /pKK-epd, *E. coli* AT1024 /pKK-serC, *E. coli* AT1024/pKK-dxs, *E. coli* AT1024 /pKK-pdxB, *E. coli* AT1024/pKK-pdxJ and *E. coli* AT1024/pKK-pdxA were prepared by transformation of plasmids pKK-epd, pKK-serC, pKK-dxs, pKK-pdxB, pKK-pdxJ and pKK-pdxA, respectively, into *E. coli* AT1024. *E. coli* AT1024/pKK-pdxJ and pVK-dxs was prepared by transformation of plasmid pVK-dxs into *E*. *coli* AT1024/pKK-pdxJ. *E. coli* AT1024/pKK-epd and pVK-pdxJ was prepared by transformation of plasmid pVK-pdxJ into *E*. *coli* AT1024/pKK-epd. *E. coli* AT1024/pKK-epd and pVK-dxs was prepared by transformation of plasmid pVK-dxs into *E. coli* AT1024/pKK-epd. *E. coli* AT1024/pKK-epd, pVK-pdxJ and pSTV-dxs was prepared by transformation of plasmid pSTV-dxs into *E. coli* AT1024/ pKK-epd and pVK-pdxJ. Each recombinant strain was stored as frozen stock made as follows. Each recombinant strain was cultured overnight in liquid LB medium with appropriate antibiotics. Cells were harvested, washed with saline, suspended in sterile 15 % glycerol solution at OD₆₀₀=5 and stored in a deep freezer at-120°C. When needed, the frozen stock was thawed before use.

### Example 11: Production of vitamin B₆ by recombinant E. coli

Recombinant *E*. *coli* strains were cultured as follows. Each 45 µl of these frozen stock prepared in Example 10 was inoculated into a tube containing 5 ml of seed medium [10 g/L of glycerol, 10 g/L of Bacto Tryptone, 5 g/L of Bacto Yeast extract, 5 g/L of NaCl (pH not adjusted)] containing appropriate antibiotics. After shaking tubes for 16 hours at 37°C, each 0.1 ml of the culture was transferred into a flask containing 50 ml of PY30 medium (20 g/L of glycerol, 10 g/L of Bacto Tryptone, 5 g/L of Bacto Yeast extract, 5 g/L of NaCl, 200 mg/L of MgSO₄·7H₂O, 10 mg/L of FeSO₄·7H₂O, 10 mg/L of MnSO₄·5H₂O, pH6.8) with appropriate antibiotics. Flasks were shaken at 37°C at 180 rpm. After cultivation for 31 hours, amount of vitamin B₆ in supernatant of the culture broth was assayed by the turbidity method with *S*. *carlsbergensis* ATCC 9080 as described below. The supernatants of culture broth and standard solutions of pyridoxol hydrochloride (0-100 mg per liter) were serially diluted to 2.09 × 10⁻⁴ in distilled water. 100 µl of the diluted solution, 1.5 ml of distilled water and 40 µl of 1N H₂SO₄ were added to tubes in this order. After autoclaving at 120°C for 20 min, 1.5 ml of sterilized assay medium for vitamin B₆ (Nissui Seiyaku Co., Japan) containing *S*. *carlsbergensis* ATCC 9080 at OD₆₀₀=0.028 was added to the tubes. The tubes were placed with an incline of 30° and incubated without shaking at 28°C for 17 hours. The growth of cells was stopped by adding 5 ml of 0.2 N hydrochloric acid, and then the absorbance of the samples was measured at 660 nm with UV-2200 spectrophotometer (shimadzu Co. Ltd., Japan). The amount of vitamin B₆ in a sample was determined by comparing the turbidity of the sample with standard growth curve of *S*. *carlsbergensis* ATCC 9080.

Concentrations of vitamin B₆ produced with recombinant *E. coli* strains are shown in Table 1. Among recombinant *E. coli* strains carrying single extra plasmid, only three strains showed increased level of vitamin B₆. Namely, the recombinant strains harboring the pKK-epd plasmid, the pKK-pdxJ plasmid, and the pKK-dxs plasmid accumulated vitamin B₆ at 14.2 mg/L, 5.1 mg/L, and 3.9 mg/L, respectively; these are 7.1-fold, 2.55-fold, and 1.95-fold higher than vitamin B₆ accumulation of the host strain, *E*. *coli* AT1024. Further, amount of accumulated vitamin B₆ could be raised to 49.2 mg/L or 57.9 mg/L by introduction of two plasmids according to the invention, pKK-epd and pVK-dxs, or pKK-epd and pVK-pdxJ, whereas the amount of accumulated vitamin B₆ was 5.4 mg/L by introduction of pKK-pdxJ and pVK-dxs. In other words, among recombinant *E. coli* strains carrying a combination of two extra plasmids, only the combination which contains epd showed marked increase in the amount of vitamin B₆, suggesting the synergistic effects of the combination containing epd. Moreover, by introduction of three plasmids, pKK-epd, pVK-pdxJ and pSTV-dxs into one host cell, amount of vitamin B₆ was raised further to 78.5 mg/L. This corresponds to an increase of 39.3-fold compared with host strain.

These results show synergistic effects of introducing epd, pdxJ, and dxs into one host cell on vitamin B₆ production in *E. coli* AT1024.

**Table I**

| microorganism | vitamin B₆ (mg/L) | factor of increase |
|---|---|---|
| *E. coli* AT1024 | 2.0 | 1.0 |
| *E. coli* AT1024/pKK-serC | 1.3 | 0.65 |
| *E*. *coli* AT1024/pKK-pdxA | 1.5 | 0.75 |
| *E. coli* AT1024/pKK-pdxB | 2.0 | 1.0 |
| *E*. *coli* AT1024/pKK-dxs | 3.9 | 1.95 |
| *E. coli* AT1024/pKK-pdxJ | 5.1 | 2.55 |
| *E. coli* AT1024/pKK-epd | 14.2 | 7.1 |
| *E. coli* AT1024/pKK-pdxJ/pVK-dxs | 5.4 | 2.7 |
| *E. coli* AT1024/pKK-epd/pVK-pdxJ | 57.9 | 28.9 |
| *E. coli* AT1024/pKK-epd/pVK-dxs | 49.2 | 24.6 |
| *E. coli* AT1024/pKK-epd/pVK-pdxJ/pSTV-dxs | 78.5 | 39.3 |

### Example 12: Separation of vitamin B₆ from cultural broth

Produced vitamin B₆ was recovered from the culture broth of *E*. *coli* AT1024/pKK-epd, pVK-pdxJ and pSTV-dxs prepared in the same cultural conditions as described in Example 11. Pyridoxol at each purification step and the concentration was followed by HPLC as described below. 50 µl of the solution containing 100 mg/l of 4'-deoxypyridoxol hydrochloride as internal substance was added to 200 µl of the standard solutions of pyridoxol hydrochloride or the sample, and then the mixture was analyzed as follows. The analytical conditions were: column: Capcell pak C18 SG120 (4.6 × 250 mm) (Shiseido Co., Ltd., Tokyo, Japan); mobile phase: 0.1 M sodium perchlorate, 0.1 M potassium phosphate, and 2% acetonitrile (pH 3.5); column temperature: 25-26°C; flow rate: 1.0 ml/min; and detector: ultraviolet (referred to as UV hereinafter) (at 292 nm).

Two liters of the 31-hour culture broth containing 78.7 mg/L of vitamin B₆ was centrifuged at 7,500 rpm for 10 min. The pH of the resultant supernatant was adjusted to 3.1 with 1N hydrochloric acid, and then the supernatant was applied to a column (5.5 × 15 cm) packed with 350 ml of Amberlite CG 120 (H⁺ form, 100-200 mesh, Rohm and Haas Company, Philadelphia, Pennsylvania, USA). The column was washed with 500 ml of deionized water and then eluted with 5% ammonium hydroxide. The vitamin B₆ fractions were concentrated under reduced pressure. The residue thus obtained was dissolved in 10 ml of deionized water, and the solution was charged on a column (5.5 × 16 cm) packed with 380 ml of Dowex 1 × 4 (OH⁻ form, 200-400 mesh, Dow Chemical Co., Ltd., Midland, Michigan, USA), and then washed with 500 ml of deionized water. The column was then eluted with 0.1 N HCl. The fractions containing pyridoxol were concentrated to small volume under reduced pressure. After the solid residue was dissolved in a small amount of hot ethanol, the solution was kept standing at 4°C overnight. The resultant precipitates were collected by filtration and dried in vacuum to obtain 128 mg of crude crystals. It was recrystallized from ethanol to obtain 98 mg of white crystals having a melting point of 160°C. The infrared absorption, UV absorption, and NMR spectrum of the product of the product coincided with those of authentic pyridoxol.

## Claims

1. A recombinant microorganism being capable of producing vitamin B₆, wherein said microorganism carries extra genes which code for an enzyme combination selected from:
i) erythrose 4-phosphate dehydrogenase and 1-deoxy-D-xylulose 5-phosphate synthase;
ii) erythrose 4-phosphate dehydrogenase and pyridoxol 5'-phosphate synthase; and
iii) erythrose 4-phosphate dehydrogenase, 1-deoxy-D-xylulose 5-phosphate synthase and pyridoxol 5'-phosphate synthase.

2. The microorganism according to claim 1, wherein said microorganism belongs to the genus *Escherichia.*

3. A process for preparing vitamin B₆ comprising the steps of:
i) culturing the recombinant microorganism of claim 1 in a fermentation broth; and
ii) separating the resulting vitamin B₆ from the fermentation broth.

4. A process for preparing vitamin B₆ comprising the steps of:
i) culturing a recombinant microorganism carrying an extra gene encoding erythrose 4-phosphate dehydrogenase in expressible form, in a fermentation broth; and
ii) separating the resulting vitamin B₆ from the fermentation broth.

5. The process according to claim 4, wherein said microorganism belongs to the genus *Escherichia.*

6. The process according to any one of claims 3 to 5, wherein said microorganism is cultured in a medium containing an assimilable carbon source, a digestible nitrogen source, inorganic salts, and other nutrients necessary for the growth of the microorganism at a pH value in the range of about 5.0 to 9.0, at a temperature in the range of from 10°C to 40°C, and for 1 day to 7 days under aerobic conditions.

## Patentansprüche

1. Rekombinanter Mikroorganismus, der Vitamin B₆ produzieren kann, wobei der Mikroorganismus Extragene trägt, die eine Enzymkombination codieren ausgewählt aus:
(i) Erythrose-4-Phosphat-Dehydrogenase und 1-Desoxy-D-Xylulose-5-Phosphat-Synthase;
(ii) Erythrose-4-Phosphat-Dehydrogenase und Pyridoxol-5'-Phosphat-Synthase und
(iii) Erythrose-4-Phosphat-Dehydrogenase, 1-Desoxy-D-Xylulose-5-Phosphat-Synthase und Pyridoxol-5'-Phosphat-Synthase.

2. Mikroorganismus nach Anspruch 1, wobei der Mikroorganismus zur Gattung *Escherichia* gehört.

3. Verfahren zur Herstellung von Vitamin B₆, das die Stufen umfasst:
(i) dass der rekombinante Mikroorganismus von Anspruch 1 in einer Fermentationsbrühe gezüchtet wird und
(ii) das entstehende Vitamin B₆ aus der Fermentationsbrühe abgetrennt wird.

4. Verfahren zur Herstellung von Vitamin B₆, das die Stufen umfasst:
(i) dass ein rekombinanter Mikroorganismus, der ein Extragen trägt, das Erythrose-4-Phosphat-Dehydrogenase in exprimierbarer Form codiert, in einer Fermentationsbrühe gezüchtet wird und
(ii) das entstehende Vitamin B₆ aus der Fermentationsbrühe abgetrennt wird.

5. Verfahren nach Anspruch 4, wobei der Mikroorganismus zur Gattung *Escherichia* gehört.

6. Verfahren nach einem der Ansprüche 3 bis 5, wobei der Mikroorganismus in einem Medium, das eine assimilierbare Kohlenstoffquelle, eine verdaubare Stickstoffquelle, anorganische Salze und andere Nährstoffe, die für das Wachstum des Mikroorganismus notwendig sind, enthält, bei einem pH-Wert im Bereich von etwa 5,0 bis 9,0, bei einer Temperatur im Bereich von 10°C bis 40°C 1 bis 7 Tage lang unter aeroben Bedingungen gezüchtet wird.

## Revendications

1. Micro-organisme recombinant pouvant produire de la vitamine B₆, dans lequel ledit micro-organisme comporte des gènes supplémentaires qui codent pour une combinaison d'enzymes choisie parmi :
i) l'érythrose 4-phosphate déshydrogénase et la 1-désoxy-D-xylulose 5-phosphate synthase;
ii) l'érythrose 4-phosphate déshydrogénase et la pyridoxol 5'-phosphate synthase; et
iii) l'érythrose 4-phosphate déshydrogénase, la 1-désoxy-D-xylulose 5-phosphate synthase et la pyridoxol 5'-phosphate synthase.

2. Micro-organisme suivant la revendication 1, dans lequel ledit micro-organisme appartient au genre *Escherichia.*

3. Procédé de préparation de vitamine B₆ comprenant les étapes de :
i) culture du micro-organisme recombinant de la revendication 1 dans un bouillon de fermentation; et
ii) séparation de la vitamine B₆ résultante du bouillon de fermentation.

4. Procédé de préparation de vitamine B₆ comprenant les étapes de :
i) culture d'un micro-organisme recombinant comportant un gène supplémentaire codant pour l'érythrose 4-phosphate déshydrogénase sous forme exprimable, dans un bouillon de fermentation; et
ii) séparation de la vitamine B₆ résultante du bouillon de fermentation.

5. Procédé suivant la revendication 4, dans lequel ledit micro-organisme appartient au genre *Escherichia.*

6. Procédé suivant l'une quelconque des revendications 3 à 5, dans lequel le micro-organisme précité est cultivé dans un milieu contenant une source de carbone assimilable, une source d'azote digestible, des sels inorganiques et d'autres agents nutritifs nécessaires à la croissance du micro-organisme à une valeur de pH allant d'environ 5,0 à 9,0, à une température allant de 10°C à 40°C, et pendant 1 jour à 7 jours sous des conditions aérobies.
